(19) Europäisches Patentamt
European Patent Office
Office européen des brevets

(11) EP 1 498 111 A2

(12) # DEMANDE DE BREVET EUROPEEN

(43) Date de publication:
**19.01.2005 Bulletin 2005/03**

(51) Int Cl.$^7$: **A61K 7/13**, A61K 7/06

(21) Numéro de dépôt: **04291775.7**

(22) Date de dépôt: **12.07.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **16.07.2003 FR 0308675**

(71) Demandeur: **L'OREAL**
**75008 Paris (FR)**

(72) Inventeurs:
- **Rollat-Corvol, Isabelle**
**75017 Paris (FR)**
- **Samain, Henri**
**91570 Bievres (FR)**

(74) Mandataire: **Wattremez, Catherine**
**L'OREAL - D.I.P.I.**
**25-29 Quai Aulagnier**
**92600 Asnières (FR)**

(54) **Composition comprenant au moins un polymère conducteur et au moins un colorant d'oxydation et procédé la mettant en oeuvre**

(57) L'invention concerne une composition comprenant, dans un milieu cosmétiquement acceptable, (a) au moins un colorant d'oxydation, (b) au moins un polymère conducteur de préférence comprenant au moins une unité répétitive de type des anilines, pyrroles, thiophènes ou bisthiophènes, furanes, para-phénylène-sulfures, paraphénylène vinylènes, indoles, amides aromatiques, hydrazides aromatiques, azométhines aromatiques, esters aromatiques particuliers.
Elle concerne de plus une composition prête à l'emploi comprenant la composition précitée et un procédé mettant en oeuvre une telle composition prête à l'emploi, ainsi que l'utilisation de cette composition pour conférer un effet optique aux fibres kératiniques.

EP 1 498 111 A2

## Description

L'invention concerne une composition comprenant, dans un milieu cosmétiquement acceptable, au moins un polymère conducteur et au moins un colorant d'oxydation. Elle concerne également un procédé de traitement de fibres kératiniques mettant en oeuvre la composition précitée. Elle a enfin pour objet l'utilisation d'une telle composition afin d'apporter un effet optique aux fibres kératiniques.

La présente invention a trait au domaine de la coloration capillaire et plus particulièrement à des colorations dites permanentes obtenues au moyen de colorants d'oxydation.

Les colorants d'oxydation sont habituellement constitués d'au moins un précurseur de colorant d'oxydation (appelé aussi base d'oxydation) éventuellement associé à au moins un coupleur qui permet de faire varier la nuance obtenue avec le ou les précurseurs.

Généralement, les précurseurs de colorant d'oxydation, sont choisis parmi les des ortho- ou para-phénylènediamines, les ortho- ou para-aminophénols, des composés hétérocycliques comme notamment les dérivés de diaminopyrazole. Ce sont des composés incolores ou faiblement colorés qui, associés à des produits oxydants, conduisent par un processus de condensation oxydative à des composés colorés.
Habituellement les coupleurs sont choisis parmi les méta-diamines aromatiques, les méta-aminophénols, les méta-diphénols et certains composés hétérocycliques tels que des composés indoliques, entre autres.

Il n'est pas rare, dans le but d'améliorer les effets optiques apportés aux fibres kératiniques après le procédé de coloration, comme des effets de brillance par exemple, d'incorporer dans la composition, des agents particuliers.
Par exemple, pour donner de la brillance aux fibres, on utilise notamment des substances hydrophobes lubrifiantes, telle que des huiles ou des cires organiques ou des silicones. Toutefois, l'effet de brillance obtenu manque d'intensité et leur donne en général un aspect artificiel.
En outre, de telles compositions présentent l'inconvénient d'apporter un toucher gras ou collant aux fibres.

Enfin, la présence de composés de ce type peut limiter la montée du colorant dans les fibres et par conséquent donner des colorations moins intenses.

La présente invention a donc pour but de proposer des compositions comprenant au moins un colorant d'oxydation, qui apportent aux fibres kératiniques traitées un effet optique particulier sans les inconvénients rencontrés avec les compositions classiques, tout en conservant de bonnes propriétés de coloration, comme des couleurs tenaces, intenses, peu sélectives.

Enfin, les fibres présentent de manière avantageuse un toucher doux et agréable.

La présente invention a ainsi pour premier objet une composition comprenant, dans un milieu cosmétiquement acceptable,

(a) au moins un colorant d'oxydation,
(b) au moins un polymère conducteur.

Un autre objet de l'invention est constitué par une composition prête à l'emploi comprenant la composition selon l'invention, et au moins un agent oxydant.

L'invention concerne de plus un procédé de traitement des fibres kératiniques, en particulier humaines, et plus particulièrement des cheveux, consistant à mettre en oeuvre les étapes suivantes :

a) on applique sur les fibres sèches ou humides, ladite composition prête à l'emploi et on laisse pauser pendant une durée suffisante pour développer la coloration ;
b) on rince éventuellement les fibres,
c) on lave et on rince les fibres,
d) on sèche ou on laisse sécher lesdites fibres.

La présente invention a de même pour objet l'utilisation d'une composition comprenant, au moins un polymère conducteur et au moins un colorant d'oxydation pour conférer un effet optique aux fibres kératiniques.

La composition selon l'invention apporte en effet à l'ensemble des fibres, et de façon uniforme, un effet optique particulier, et notamment une brillance plus intense, plus naturelle, et plus esthétique qu'avec les moyens de l'art antérieur.

Par ailleurs, lorsque les polymères conducteurs présents dans la composition selon l'invention absorbent dans le spectre visible, on obtient simultanément un effet optique, comme de la brillance, et de la couleur.
Cela peut aussi permettre d'élargir la palette des couleurs susceptibles d'être obtenues, ou bien encore d'optimiser les teneurs en colorants d'oxydation.

Mais d'autres caractéristiques et avantages de l'invention apparaîtront plus clairement à la lecture de la description et des exemples qui suivent.

Dans ce qui va suivre et à moins d'une indication différente, les bornes d'un domaine de valeurs sont comprises comme faisant partie de ce domaine.
Au sens de la présente invention, le terme effet optique recouvre des effets de brillance, de couleur, métallique, goniochromatique, moiré.
Par ailleurs, et plus particulièrement, il est à noter que la brillance correspond à l'intensité lumineuse réfléchie sous un angle $\alpha$ lorsque la mèche de cheveux est éclairée sous un angle -$\alpha$. L'angle $\alpha$ classiquement utilisé pour mesurer cette réflexion spéculaire, autrement dit la brillance, est égal à 20°. Cet apport de brillance peut être mesuré par utilisation d'un brillancemètre comme il est par exemple décrit dans la norme ISO 2813 - 1994 de l' AFNOR (août 1994, rectificatif février 1997).

Polymères conducteurs

Selon la présente invention, on entend par "polymère conducteur" une structure moléculaire dans laquelle le ou les monomères présentent une forte délocalisation électronique et dont la disposition dans le squelette du polymère permet aux orbitales $\pi$ de se recouvrir. Cette caractéristique chimique se traduit par un phénomène de conduction électrique qui s'accompagne ou non d'un phénomène d'absorption dans le spectre UV-visible, voire dans l'infrarouge.
Par polymère conducteur absorbant dans le visible, on entend au sens de la présente invention, tout polymère conducteur présentant une absorbance non nulle dans le domaine de longueur d'ondes allant de 400 à 800 nm, même si les maxima d'absorption du polymère se situent en dehors de cette gamme.
Les polymères conducteurs mis en oeuvre dans le cadre de la présente invention sont des polymères conducteurs solubles ou dispersibles dans le milieu cosmétique approprié à l'application.
On dit que le polymère est soluble dans le milieu lorsqu'il forme un liquide limpide isotrope à 25°C dans le milieu comprenant de l'eau ou un mélange eau/solvant ; ceci étant obtenu dans tout ou partie d'une gamme de concentration comprise entre 0,01 et 50 % en poids de polymère conducteur.
De façon préférée, les polymères conducteurs mis en oeuvre dans le cadre de la présente invention sont des polymères conducteurs solubles ou dispersibles dans un milieu aqueux, avantageusement dans l'eau.
On dit que le polymère est dispersible dans le milieu comprenant de l'eau ou un mélange eau/solvant si, à 0,01% en poids, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.
Il est à noter que de manière avantageuse, ces polymères ne nécessitent pas l'emploi d'un agent dispersant.
De préférence, les polymères conducteurs se présentent sous une forme soluble dans le milieu de la composition.
De plus, les polymères présentent de manière avantageuse, une conductivité comprise entre $10^{-5}$ et $5.10^{5}$ siemens/cm, plus particulièrement comprise entre $10^{-3}$ et $10^{5}$ siemens/cm, et de préférence comprise entre $10^{-1}$ et $10^{4}$ siemens/cm.
La conductivité est mesurée à l'aide d'un générateur de courant (RM2 Test Unit commercialisé par la société Jandel) muni d'une tête de mesure dite quatre pointes (Universal four-point probes commercialisé par la société Jandel). Les quatre pointes alignées et distantes du même espacement d sont appliquées par simple pression sur l'échantillon à analyser. Un courant I est injecté par les pointes externes à l'aide de la source de courant créant ainsi une variation de potentiel. La tension U est mesurée entre les deux pointes internes reliées au voltmètre du générateur de courant.
Dans cette configuration la conductivité de l'échantillon exprimée en S/cm est donnée par l'expression suivante :

$$\sigma = (K \times I) / (U \times e)$$

Avec :

K coefficient dépendant de la position des contacts sur la surface de l'échantillon.
Lorsque les pointes sont alignées et équidistantes, K est égal à : $\Pi/\log(2)$
I : valeur du courant injecté exprimé en ampères
U : valeur de la tension mesurée exprimée en volts
e : épaisseur de l'échantillon exprimée en cm

Cette expression ne peut être utilisée que lorsque l'épaisseur du matériau est négligeable devant la distance d existant entre deux pointes (e/d < 0,25). Pour obtenir des épaisseurs suffisamment faibles et ainsi pouvoir calculer la conductivité du matériau, il est préconisé de réaliser la mesure sur un support non conducteur (par exemple, une

lame de verre) recouvert du matériau à analyser obtenu par évaporation d'une solution diluée. Afin d'améliorer l'homogénéité du revêtement à analyser, il est également préconisé d'utiliser la technique de dépôt dite du spin coating.

Selon un mode de réalisation particulier, les polymères conducteurs présents dans la composition sont choisis parmi les polymères comprenant au moins une unité répétitive de formules suivantes :

les anilines de structure (I) suivante :

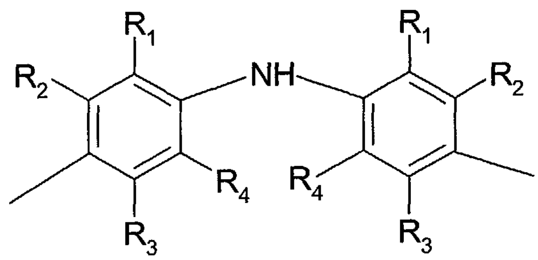

(I)

les pyrroles de structure (IIa) et (IIb) suivantes :

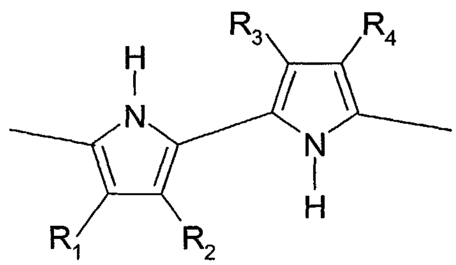

(IIa)

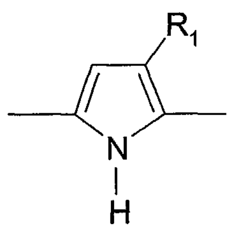

(IIb)

les thiophènes ou bisthiophènes de formules (IIIa), (IIIb) et (IIIc) suivantes :

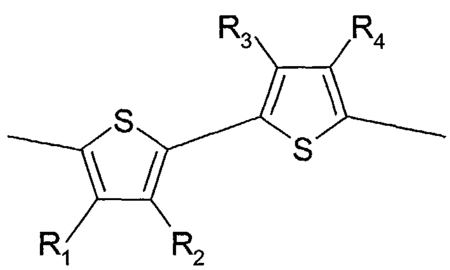

(IIIa)

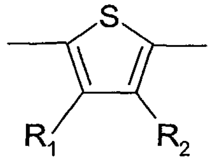

(IIIb)

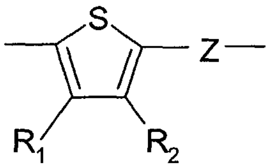

(IIIc)

les furanes de formule (IV) suivante :

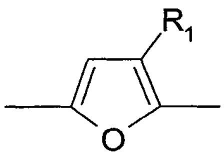

(IV)

les para-phénylène-sulfure de structure (V) suivante :

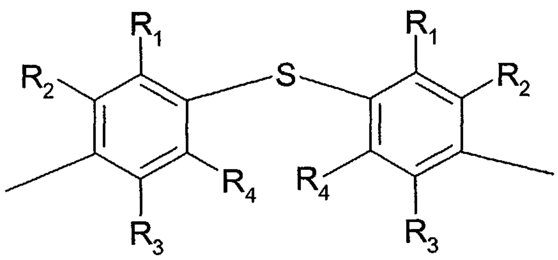

(V)

les paraphénylène vinylène de formule (VI) suivante :

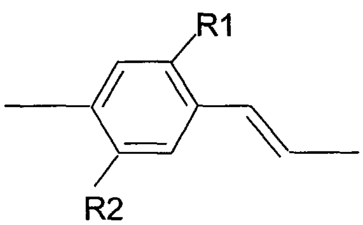

(VI)

les indoles de formule (VII) suivante :

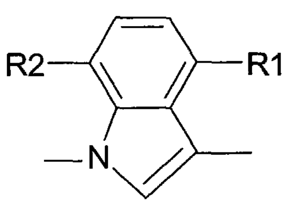

(VII)

les amides aromatiques de formules suivantes (VIIIa), (VIIIb, (VIIIc), (VIIId) :

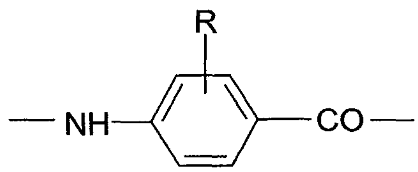

(VIIIa)

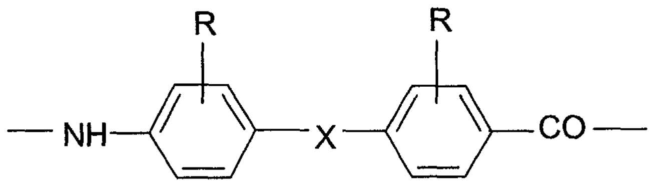

(VIIIb)

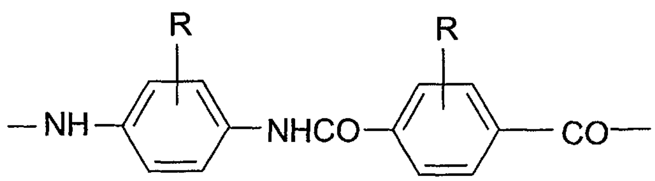

(VIIIc)

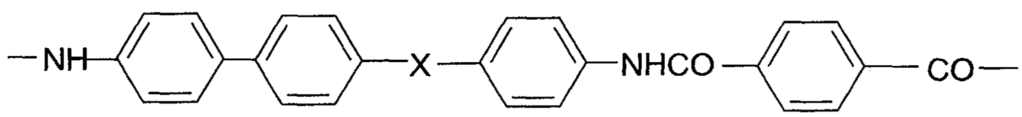

(VIIId)

les hydrazides aromatiques de formules (IXa), (IXb) et (IXc) suivantes :

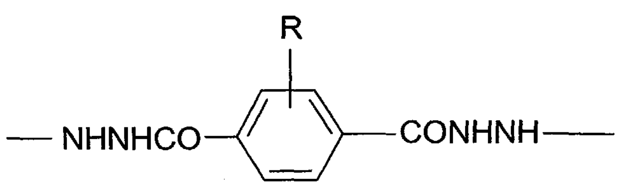

(IXa)

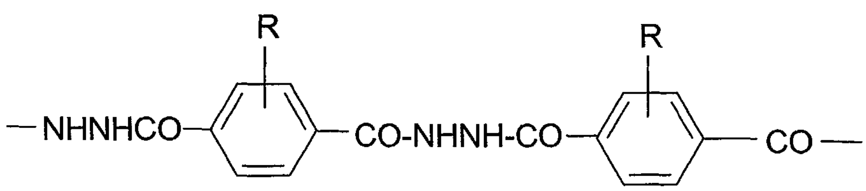

(IXb)

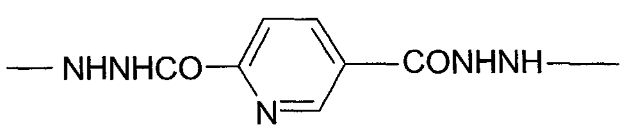

(IXc)

les azométhines aromatiques de formules (Xa), (Xb) et (Xc) suivantes :

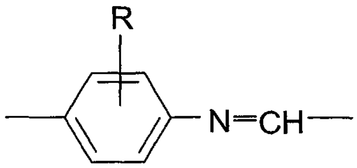

(Xa)

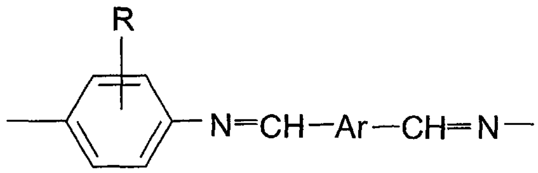

(Xb)

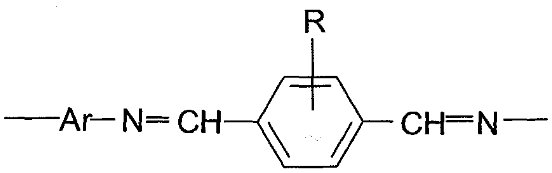

(Xc)

les esters aromatiques de formules (XIa), (XIb) et (XIc) suivantes :

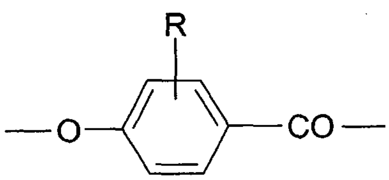

(XIa)

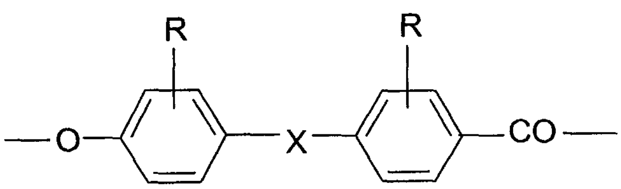

(XIb)

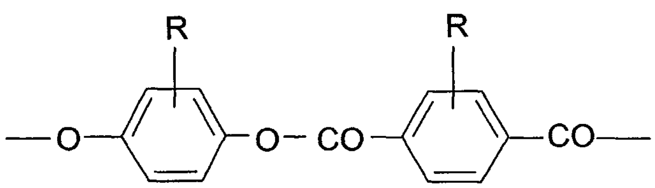

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N- ;

Z = -CH=CH- ou -C≡C-.

Plus particulièrement, Ar représente au moins un radical choisi parmi les suivants :

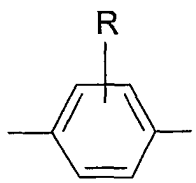
,
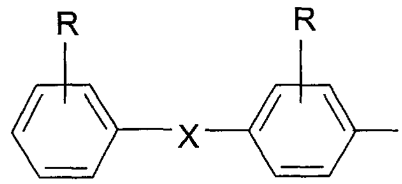
,
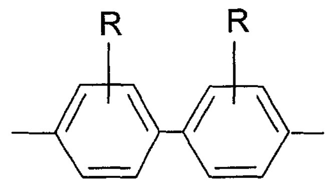
.

Par groupement solubilisant, on entend au sens de la présente invention, un groupement qui assure la solubilisation de ladite molécule dans le milieu cosmétique, de façon que le polymère présente un caractère conducteur après séchage de la composition.

Il est clair que le polymère conducteur présent dans la composition selon l'invention peut comprendre une ou plusieurs unités répétitives comprenant un ou plusieurs groupements solubilisants, et d'une ou plusieurs autres qui en sont dépourvues.

Les groupements solubilisants sont de préférence choisis dans le groupe formé par :

- un radical carboxylique (-COOH), carboxylate ($-COO-M^+$ avec M représentant un métal alcalin comme le sodium, le potassium, un métal alcalino-terreux, une amine organique telle que une amine primaire, secondaire ou tertiaire, une alcanolamine, un acide aminé),
- un radical sulfonique ($-SO_3H$), sulfonate ($-SO_3^-\ M^+$, M ayant la même définition que ci-dessus),
- un radical amine primaire, secondaire, tertiaire,
- un radical ammonium quaternaire tel $-NR'3^+\ Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

Les fonctions acides carboxyliques ou sulfoniques peuvent ou non être neutralisées par une base, telle que l'hydroxyde de sodium, l'amino-2 méthyl-2 propanol, la triéthylamine ou encore la tributylamine, par exemple.
Les radicaux amines peuvent ou non être neutralisés par un acide minéral, tel que l'acide chlorhydrique, ou par un acide organique, tel que les acides acétique ou lactique, par exemple.

En outre, il est à noter que lesdits radicaux solubilisants peuvent être reliés au cycle par l'intermédiaire d'un groupement espaceur tel que par exemple un radical -R"-, -OR"-, - OCOR"- ou encore -COOR"- avec R" représentant un radical alkyle, linéaire ou ramifié en $C_1$-$C_{20}$, comprenant éventuellement un ou plusieurs hétéroatomes, tels que l'oxygène par exemple.

De préférence les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : - COOH, $-CH_2COOH$, $-CH_2OH$, $-(CH_2)_6OH$, $-(CH_2)_3SO_3H$, $-O(CH_2)_3SO_3H$, $-O(CH_2)_3N(CH_2CH_3)_2$, $-[(CH_2)_2O]_xCH_2CH_2OH$, $-[(CH_2)_2O]_xCH_2CH_2OCH_3$ avec x nombre moyen compris entre 0 et 200.

Le nombre d'unités répétitives du polymère n varie habituellement de 5 à 10000, notamment de 5 à 1000, plus particulièrement de 10 à 1000 et de préférence de 20 à 700.

Plus particulièrement, le polymère conducteur est tel qu'au moins un radical parmi R, R1 à R4 désigne un groupement solubilisant.

Conformément à un mode de réalisation particulier de l'invention, le polymère conducteur mis en oeuvre comporte au moins un groupement solubilisant par unité répétitive. Ainsi, de préférence, au moins un radical parmi R, $R_1$ à $R_4$ désigne un groupement solubilisant.

De préférence, le polymère conducteur est soluble dans le milieu de la composition.

Les polymères conducteurs présents dans la composition selon l'invention sont bien connus de l'homme de l'art et décrits notamment dans l'ouvrage "Handbook of organic conductive molecules and polymers" - Wiley 1997- New York, Vol 1, 2, 3, mais aussi dans la revue Can. J. Chem. Vol 64, 1986.
Les polythiophènes et leur synthèse sont plus particulièrement décrits dans l'article tiré de la revue Chem. Mater. 1998, Vol.10, N°7 pages 1990-1999 - par les auteurs RASMUSSEN S.C., PICKENS J.C. et HUTCHISON J.E. "A new, general

approach to tuning the properties of functionalized polythiophenes : The oxidative polymerization of monosubstituted bithiophenes" ; dans l'article tiré de la revue Macromolecules 1998, 31, pages 933-936, par les mêmes auteurs "Highly conjugated, water-soluble polymers via direct oxidative polymerization of monosubstituted bithiophenes". Outre la polymérisation par oxydation chimique ou électrochimique, ils peuvent être aussi obtenus par polycondensation (thiophène dihalogéné ; catalyse avec des complexes de nickel ou de palladium) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polymères conducteurs présents dans la composition selon l'invention sont par ailleurs décrits dans la demande internationale WO 99/47570.

Parmi les polymères conducteurs convenables à la réalisation de la présente invention, on peut citer plus particulièrement les polymères correspondant aux formules (IIIa), (IIIb) et (IIIc) dans lesquelles les groupes solubilisants sont de préférence un groupement acide carboxylique ; un groupement acide sulfonique ; un radical amine tertiaire ; un radical ammonium quaternaire tel que $-NR'_3^+ Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux ; lesdits groupes étant éventuellement reliés au cycle par un espaceur. Les fonctions acides carboxyliques ou sulfoniques peuvent ou non être neutralisées.

Ainsi, la polymérisation peut être réalisée par oxydation chimique ou électrochimique du monomère thiophène correspondant ou bien encore par polycondensation.

A titre d'illustration, les polythiophènes de formules (IIIa) et (IIIb) peuvent être obtenus par polymérisation par oxydation (par exemple avec une catalyse $FeCl_3$); par polycondensation de thiophène dihalogéné catalysée par des complexes de nickel ou de palladium (ex. : $NiCl_2(dppe)_2$); par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B)) ; par couplage de Stille (formation de liaison carbone-carbone en présence d'une catalyse à base de Pd-type AA-BB ou A-B) ; par polymérisation de Reike (organozinc en présence d'un complexe de nickel) ; par polymérisation de type McCulloch, etc.

Les polythiophènes vinylènes de formule (IIIc) avec Z représentant -CH=CH-, peuvent notamment être obtenus par polymérisation de Gilch en présence d'une base forte (tertiobutylate de potassium), de 2,5 bis (bromoalkylène) thiophène ; par polymérisation par la méthode de Wessling via l'utilisation de précurseur à base de sels de sulfonium et pyrolyse ; par réaction de Wittig Wittig-Horner.

Les polythiophènes éthynylènes de formule (IIIc) avec Z représentant-C≡C- peuvent être obtenus par couplage de Heck-Sonogashira (de type AA-BB ou A-B ; formation de liaison carbone-carbone entre une fonction acétylénique terminale (ou acétylénique vraie) et une fonction bromo ou iodo catalysée par un complexe de palladium/cuivre ($PdCl_2(PPh_3)_3$, CuI ou $Cu(Oac)_2$) en présence d'une base telle que la triéthylamine, diisopropylamine, piperidine etc...) ; par méthathèse d'alkynes en présence d'un complexe de molybdène (de $Mo(CO)_6$).

En général la fonctionnalisation des polythiophènes, en d'autres termes, l'apport du ou des groupes solubilisants ou non, est réalisée sur le monomère avant sa polymérisation.

Dans certains cas, l'obtention du groupe solubilisant est obtenu par suite de traitement de polymère. C'est notamment le cas de la fonction acide carboxylique qui peut être obtenue par hydrolyse de l'ester correspondant.

De préférence, les groupes solubilisants sont choisis parmi les groupements acide carboxylique ; acide sulfonique ; les radicaux amine tertiaire ; ammonium quaternaire tels que $-NR'_3^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels.

Les fonctions acides carboxyliques ou sulfoniques peuvent ou non être neutralisées.

Selon un mode de réalisation particulier de l'invention, le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

Les polymères conducteurs sont généralement présents dans la composition dans des proportions d'au moins 0,001 % en poids, plus particulièrement d'au moins 0,01 % en poids, de préférence d'au moins 0,1 % en poids et de manière encore plus préférée, d'au moins 0,5 % en poids, par rapport au poids total de la composition. Par ailleurs, la teneur en polymère conducteur est avantageusement d'au plus 50 % en poids, plus particulièrement d'au plus 30 % en poids, de préférence d'au plus 20 % en poids et de manière encore plus préférée d'au plus 10 % en poids, par rapport au poids total de la composition.

Selon un mode de réalisation particulièrement avantageux de l'invention, la teneur en polymère conducteur est comprise entre 0,1 et 50% en poids, plus particulièrement entre 0,1 et 30% en poids, et de préférence entre 0,5 et 10 % en poids, par rapport au poids total de la composition.

Colorants d'oxydation

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation sont choisies parmi celles classiquement connues en teinture d'oxydation, parmi lesquelles on peut notamment citer les ortho- et para-phénylènediamines, les bases doubles, les ortho- et para- aminophénols, les bases hétérocycliques suivantes ainsi que leurs sels d'addition avec un acide.

Plus particulièrement, on peut citer :

- (I) les paraphénylènediamines de formule (A) suivante et leurs sels d'addition avec un acide :

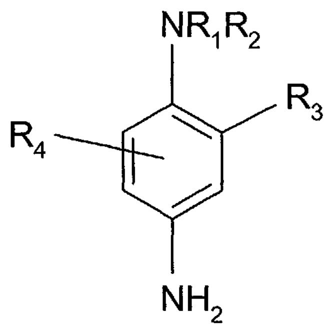

(A)

dans laquelle :

$R_1$ représente un atome d'hydrogène, un radical alkyle en $C_1$-$C_4$, monohydroxyalkyle en $C_1$-$C_4$, polyhydroxyalkyle en $C_2$-$C_4$ , alcoxy($C_1$-$C_4$)alkyle($C_1$-$C_4$), alkyle en $C_1$-$C_4$ substitué par un groupement azoté, phényle ou 4'-aminophényle ;

$R_2$ représente un atome d'hydrogène, un radical alkyle en $C_1$-$C_4$, monohydroxyalkyle en $C_1$-$C_4$ ou polyhydroxyalkyle en $C_2$-$C_4$, alcoxy($C_1$-$C_4$)alkyle($C_1$-$C_4$) ou alkyle en $C_1$-$C_4$ substitué par un groupement azoté ;

$R_1$ et $R_2$ peuvent également former avec l'atome d'azote qui les porte un hétérocycle azoté à 5 ou 6 chaînons éventuellement substitué par un ou plusieurs groupements alkyle, hydroxy ou uréido;

$R_3$ représente un atome d'hydrogène, un atome d'halogène tel qu'un atome de chlore, un radical alkyle en $C_1$-$C_4$, sulfo, carboxy, monohydroxyalkyle en $C_1$-$C_4$ ou hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,

$R_4$ représente un atome d'hydrogène, d'halogène ou un radical alkyle en $C_1$-$C_4$.

Parmi les groupements azotés de la formule (A) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (A) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthylaniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl paraphénylènediamine, la N-(β-hydroxypropyl) paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxyparaphénylènediamine, la 2-β-acétylaminoéthyloxy-paraphénylène-diamine, la N-(β-méthoxyéthyl)-paraphénylènediamine, 2-méthyl-1-N-β-hydroxyéthyl-paraphénylènediamine, et leurs sels d'addition avec un

acide.

Parmi les paraphénylènediamines de formule (A) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxy-éthyloxy paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxy-éthyl) paraphénylènediamine, la 2-chloro-paraphénylènediamine, et leurs sels d'addition avec un acide.

- (II) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (B) suivante, et leurs sels d'addition avec un acide :

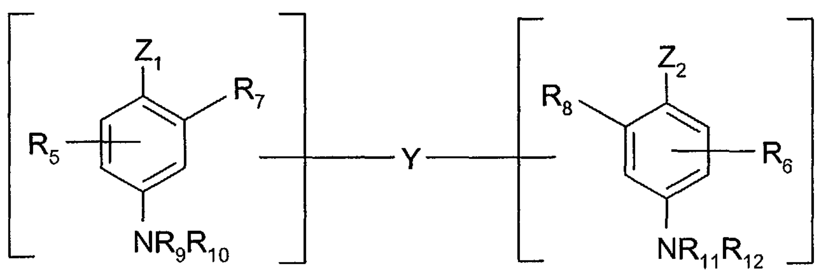

(B)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$, $R_{11}$ et $R_{12}$, identiques ou différents, représentent un atome d'hydrogène, un bras de liaison Y ou un radical alkyle en $C_1$-$C_4$;

étant entendu que les composés de formule (B) ne comportent qu'un seul bras de liaison Y par molécule.

Parmi les groupements azotés de la formule (B) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (B) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétra-méthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diamino-phénoxy)-3,5-dioxaoctane, et leurs sels d'addition avec un acide.

Parmi ces bases doubles de formule (B), le N,N'-bis-(β-hydroxyéthyl)-N,N'-bis-(4'-aminophényl)-1,3-diaminopropanol, le 1,8-bis-(2,5-diaminophénoxy)-3,5-dioxaoctane ou l'un de leurs sels d'addition avec un acide sont particulièrement préférés.

- (III) les para-aminophénols répondant à la formule (C) suivante, et leurs sels d'addition avec un acide :

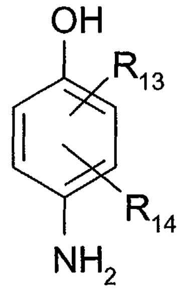

(C)

dans laquelle :

$R_{13}$ représente un atome d'hydrogène,un atome d'halogène tel que le fluor, un radical alkyle en $C_1$-$C_4$, monohydroxyalkyle en $C_1$-$C_4$, alcoxy($C_1$-$C_4$)alkyle($C_1$-$C_4$) ou aminoalkyle en $C_1$-$C_4$, ou hydroxyalkyl($C_1$-$C_4$)aminoalkyle en $C_1$-$C_4$.

$R_{14}$ représente un atome d'hydrogène ou un atome d'halogène tel que le fluor, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$).

Parmi les para-aminophénols de formule (C) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthylphénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

- (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, la 2-(4-méthoxyphényl)amino-3-aminopyridine, la 2,3-diamino-6-méthoxypyridine, la 2-(β-méthoxyéthyl)amino-3-amino-6-méthoxy pyridine, la 3,4-diaminopyridine, et leurs sels d'addition avec un acide.

Parmi les dérivés pyrimidiniques, on peut plus particulièrement citer les composés décrits par exemple dans les brevets et demandes de brevets DE 2359399, JP 88-169571, JP 91-10659, WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR 2750048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets et demandes de brevets DE 3843892, DE 4133957, WO 94/08969, WO 94/08970, FR 2733749 et DE 19543988, comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthylpyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent plus particulièrement de 0,0005 à 12% en poids par rapport au poids de la composition et de préférence de 0,005 à 8% en poids par rapport au poids de la composition.

Les coupleurs utilisables dans la composition sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthylbenzène, le 4-chloro-1,3-dihydroxy-benzène, le 2-amino 4-(β-hydroxyéthylamino)-1-méthoxybenzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthylpyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

<u>Tensioactifs</u>

De préférence, la composition cosmétique selon l'invention comprend un ou plusieurs tensioactifs qui peuvent être indifféremment choisis, seuls ou en mélanges, parmi des tensioactifs anioniques, amphotères, non ioniques, zwittérioniques et cationiques.

Les tensioactifs convenant à la mise en oeuvre de la présente invention sont notamment les suivants :

(i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl ($C_6$-$C_{24}$) sulfosuccinates, les alkyl($C_6$-$C_{24}$) éthersulfosuccinates, les alkyl($C_6$-$C_{24}$) amidesulfosuccinates ; les alkyl ($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent

être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

(iii) Tensioactif(s) amphotère(s) ou zwittérionique(s) :

Les agents tensioactifs amphotères ou zwitterioniques peuvent être notamment choisis parmi les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C8-C20) bétaïnes, les sulfobétaïnes, les alkyl (C8-C20) amidoalkyl (C1-C6) betaïnes ou les alkyl (C8-C20) amidoalkyl (C1-C6) sulfobétaïnes.

Parmi les dérivés d'amines, on peut citer les produits vendus sous la dénomination MIRANOL, tels que décrits dans les brevets US 2528378 et US 2781354 et classés dans le dictionnaire CTFA, 3ème édition, 1982, sous les dénominations Amphocarboxyglycinates et Amphocarboxypropionates de structures respectives :

$$Rd\ \text{-CONHCH2CH2 -N(Re)(Rf)(CH2COO}^-\text{)}$$

dans laquelle : Rd désigne un radical alkyle d'un acide Rd-COOH présent dans l'huile de coprah hydrolysée, un radical heptyle, nonyle ou undécyle, Re désigne un groupement bêta-hydroxyéthyle et Rf un groupement carboxyméthyle ; et

$$\text{Rg-CONHCH2CH2-N(B)(C)}$$

dans laquelle :

B représente -CH2CH2OX, C représente -(CH2)z -Y, avec z = 1 ou 2,
X désigne le groupement -CH2CH2-COOH ou un atome d'hydrogène
Y désigne -COOH ou le radical -CH2 - CHOH - SO3H
Rg désigne un radical alkyle d'un acide Rh -COOH présent dans l'huile de coprah ou dans l'huile de lin hydrolysée, un radical saturé ou comprenant une ou plusieurs insaturations, notamment en C7 à C17, plus particulièrement un radical alkyle en C9, C11, C13, C17 ou sa forme iso, un radical C17 insaturé.

Ces composés sont classés dans le dictionnaire CTFA, 5ème édition, 1993, sous les dénominations Disodium Coco-ampho-diacetate, Disodium Lauro-ampho-diacetate, Disodium Capryl-ampho-diacetate, Disodium Caprylo-ampho-diacetate, Disodium Coco-ampho-dipropionate, Disodium Lauro-ampho-dipropionate, Disodium Capryl-ampho-dipropionate, Disodium Caprylo-ampho-dipropionate, Lauro-ampho-dipropionic acid, Coco-ampho-dipropionic acid. A titre d'exemple on peut citer le cocoamphodiacetate commercialisé sous la dénomination commerciale Miranol® C2M concentré par la société Rhodia Chimie.

(iv) Tensioactifs cationiques :

Parmi les tensioactifs cationiques on peut citer en particulier les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

La quantité d'agents tensioactifs présents dans la composition selon l'invention peut varier de 0,01 à 40 % en poids et de préférence de 0,5 à 30 % en poids, du poids total de la composition.

Milieu

Le milieu cosmétiquement acceptable de la composition cosmétique est de préférence un milieu aqueux constitué par de l'eau et peut avantageusement comprendre des solvants organiques acceptables sur le plan cosmétique, dont plus particulièrement, des alcools en C1-C4, tels que l'alcool éthylique, l'alcool isopropylique, les alcools aromatiques comme l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore des polyols comme la glycérine. On peut également utiliser comme solvant les polyéthylèneglycols et le polypropylèneglycol, et les mélanges de tous ces composés.
Le ou les solvants peuvent alors être présents dans des concentrations comprises entre 0,5 et 20% et, de préférence, entre 2 à 10% en poids par rapport au poids total de la composition.

Additifs

La composition cosmétique peut encore comprendre une quantité efficace d'autres agents, par ailleurs antérieurement connus dans le traitement des fibres kératiniques humaines, tels que des agents épaississants, des agents anti-oxydants, des parfums, des agents dispersants, des agents de conditionnement dont notamment des polymères cationiques ou amphotères, des agents opacifiants, des agents séquestrants tel que l'EDTA et l'acide étidronique, des filtres UV, des cires, des silicones volatiles ou non, cycliques ou linéaires ou ramifiées, organomodifiées (notamment par des groupements amines) ou non, des agents conservateurs, des céramides, des pseudocéramides, des huiles végétales, minérales ou de synthèse, les vitamines ou provitamines comme le panthénol, des polymères associatifs non-ioniques, anioniques, amphotères ou cationiques.
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.
Un autre objet de l'invention est une composition prête à l'emploi comprenant la composition qui vient d'être décrite, et au moins un agent oxydant.
L'agent oxydant est choisi par exemple parmi le peroxyde d'hydrogène, le peroxyde d'urée, les bromates ou ferricyanures de métaux alcalins, les persels tels que les perborates et persulfates, et les enzymes telles que les peroxydases et les oxydoréductases à deux ou à quatre électrons.
L'utilisation du peroxyde d'hydrogène est particulièrement préférée.
Cet agent oxydant peut être avantageusement constitué par une solution d'eau oxygénée dont le titre peut varier, plus particulièrement, d'environ 1 à 40 volumes, et encore plus préférentiellement d'environ 5 à 40.
Par ailleurs, la teneur en agent oxydant représente en général 0,001 à 10% en poids par rapport au poids de la composition prête à l'emploi.
Le pH de la composition selon l'invention ou de la composition prête à l'emploi est généralement compris entre les 4 et 12, de préférence entre 6 et 11.
Il peut être ajusté à la valeur désirée au moyen d'agents acidifiants ou alcalinisants bien connus de l'état de la technique en teinture des fibres kératiniques.
Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante :

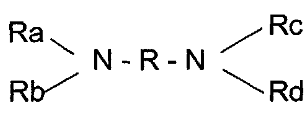

dans laquelle R est un reste propylène éventuellement substitué par un groupement hydroxyle ou un radical alkyle en $C_1$-$C_4$ ; Ra, Rb, Rc et Rd, identiques ou différents, représentent un atome d'hydrogène, un radical alkyle en $C_1$-$C_4$ ou hydroxyalkyle en $C_1$-$C_4$.
Les agents acidifiants sont classiquement, à titre d'exemples, des acides minéraux ou organiques comme l'acide chlorhydrique, l'acide orthophosphorique, des acides carboxyliques comme l'acide tartrique, l'acide citrique, l'acide lactique, ou des acides sulfoniques.

Comme indiqué auparavant, le procédé selon l'invention consiste à effectuer les étapes suivantes :

a) on applique ladite composition prête à l'emploi sur les fibres sèches ou humides, et on laisse pauser pendant une durée suffisante pour développer la coloration,
b) on rince éventuellement les fibres,
c) on lave les fibres et on les rince,
d) on sèche ou on laisse sécher les fibres.

Selon une première variante de l'invention, la composition prête à l'emploi est obtenue par mélange extemporané, juste avant l'application sur les fibres, d'une composition selon l'invention, avec une composition oxydante, comprenant dans un milieu approprié pour la teinture, au moins un agent oxydant.
L'agent oxydant présent dans la composition oxydante peut être choisi parmi les agents listés dans le cadre de la définition de la composition prête à l'emploi et l'on pourra s'y référer.
Le milieu de la composition oxydante est avantageusement de l'eau ou un mélange d'eau et de solvant organique. Là encore, la liste de ces composés indiquée dans la description de la composition selon l'invention est valable.
Enfin, cette composition peut comprendre les additifs usuels dans le domaine, comme des agents tensioactifs, des agents épaississants, des agents anti-oxydants, des parfums, des agents dispersants, des agents de conditionnement, des agents séquestrants, des agents conservateurs, etc.
Selon une deuxième variante du procédé, la composition prête à l'emploi est obtenue en appliquant successivement, dans un ordre ou dans l'autre, la composition selon l'invention et la composition oxydante.
Il est à noter que conformément à un mode de réalisation particulier, il est possible de stocker sous forme séparée, d'une part, la composition selon l'invention, et d'autre part, une composition oxydante.
On procède ensuite au mélange de la composition selon l'invention et de la composition oxydante au moment de l'emploi, avant d'appliquer ce mélange sur les fibres kératiniques ; les autres étapes précitées du procédé étant ensuite mises en oeuvre.
La durée suffisante au développement de la coloration est en général comprise entre 1 à 60 minutes, et plus préférentiellement entre 5 et 45 minutes.
Par ailleurs, de manière classique l'étape a) du procédé est effectuée à une température comprise entre 15 et 80°C, de préférence à une température comprise entre 15 et 40°C.
Une fois l'étape a) terminée, on peut éventuellement rincer les fibres, les laver avec un shampooing et les rincer, puis les sécher ou les laisser sécher entre 20 et 120°C.
Les exemples suivants illustrent l'invention sans pour autant présenter un caractère limitatif.

## EXEMPLE

### Synthèse de poly(thiophène-3- acide acétique)

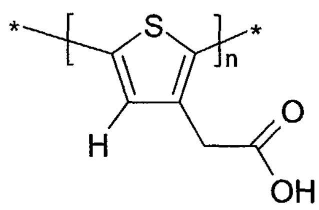

Mode opératoire

Préparation du polymère : poly(thiophène-3-acétate d'éthyle)

Dans un schlenk sous argon, on introduit 25 ml de chloroforme sec, on dégaze puis on introduit les réactifs : 2,5g de thiophène-3-acétate d'éthyle (14,7 mmol)

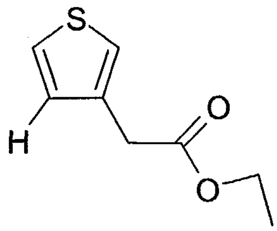

et 1 g de FeCl3 (6,15 mmol).

Le mélange est agité pendant 24 heures sous argon à 50°C.
Le polymère poly(thiophène-3-acétate d'éthyle) est alors précipité dans l'heptane.
Le polymère est ensuite dissous dans une solution de tétrahydrofuranne

Caractérisation par infra rouge :

Bande du C=O : 1719 $cm^{-1}$; bandes du CH2, CH3 = 2979 $cm^{-1}$, 2934 cm-1 et disparition de la bande CH à 3102 $cm^{-1}$ présente dans le monomère.

Hydrolyse du polymère : poly(thiophène-3-acétate d'éthyle) pour former le poly(thiophène-3- acide acétique). Le polymère obtenu précédemment est alors hydrolysé par un excès de 50 ml d'une solution aqueuse d'hydroxyde de sodium (2N) pendant 48heures à 70°C, suivi d'une acidification par HCl concentré jusqu'à précipitation du produit : poly(thiophène-3- acide acétique).

Le polymère est ensuite filtré et lavé à plusieurs reprises par de l'eau distillée afin d'éliminer les traces de catalyseur.

Caractérisation du polymère Infra-rouge :

Bande du C=O : 1740 $cm^{-1}$; COO 1580 $cm^{-1}$; OH (bande large 3000-3500 $cm^{-1}$)

Neutralisation du polymère poly(thiophène-3- acide acétique) :

Le polymère poly(thiophène-3- acide acétique) (2g) est dissous dans le tétrahydrofuranne (30g) et neutralisé à raison de 1 mol d'hydroxyde de sodium par mole d'acide carboxylique.
L'eau (30 g) est ensuite additionnée.
Le tétrahydrofuranne est évaporé.

Il est ainsi obtenu une solution aqueuse 6% de poly(thiophène-3- acide acétique) sous forme d'un sel de sodium.

| **Formulation comprenant le polymère obtenu et procédé de teinture :** | |
|---|---|
| On réalise la formule colorante suivante : | |
| Poly(thiophène-3-acide acétique) | 5 g |
| Paraphénylène diamine | 0,216 g |
| 2-Méthylaminophénol | 0,248 g |
| Ammoniaque à 20% | 9 g |
| Alcool éthylique | 15 g |
| Eau qs | 100 g |

On ajoute de l'eau oxygénée 20volumes (1 dose d'eau oxygénée pour 1 dose de formule colorante). Après mélange, le tout est appliqué sur des mèches de cheveux blancs.
Après 20 minutes d'attente, on procède à un rinçage léger et à un séchage (séchage à l'air libre).

Les mèches sont colorées en beige et présentent une brillance particulièrement élevée. Vues sous différents angles, les mèches présentent des reflets différents puisque s'ajoutent à la nuance beige, des reflets roses, jaunes ou verts.

On réalise le même essai mais avec une formule dans laquelle le poly(thiophène-3-acide acétique) n'est pas introduit.

Les mèches sont colorées en beige mais sont moins brillantes que les mèches traitées par la formulation selon l'invention. De plus, elles ne présentent pas de changement de reflets en fonction de l'angle de vue.

## Revendications

1. Composition comprenant, dans un milieu cosmétiquement acceptable,

   (a) au moins un colorant d'oxydation,
   (b) au moins un polymère conducteur.

2. Composition selon la revendication précédente **caractérisée en ce que** le polymère conducteur comprend au moins une unité répétitive de formules suivantes :

   les anilines de structure (I) suivante :

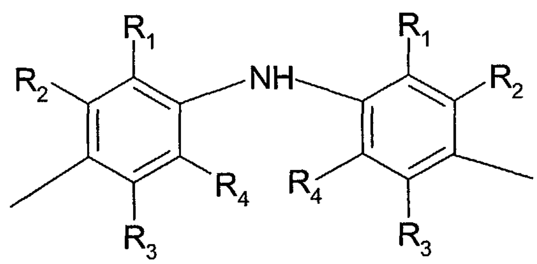

(I)

   les pyrroles de structure (IIa) et (IIb) suivantes :

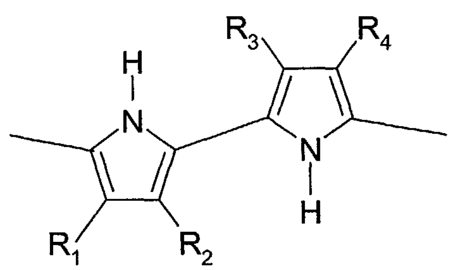

(IIa)

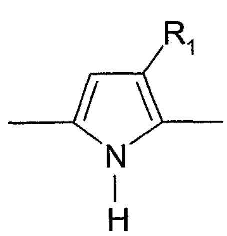

(IIb)

   les thiophènes ou bisthiophènes de formules (IIIa), (IIIb) et (IIIc) suivantes :

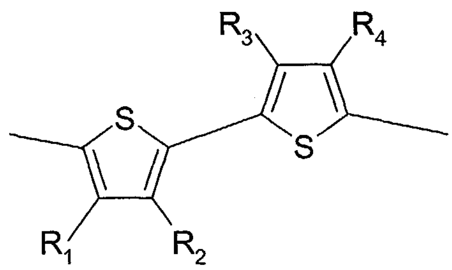

(IIIa)

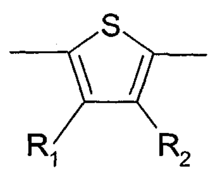

(IIIb)

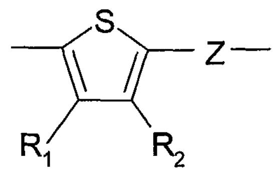

(IIIc)

les furanes de formule (IV) suivante :

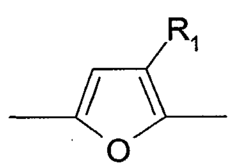

(IV)

les para-phénylène-sulfure de structure (V) suivante :

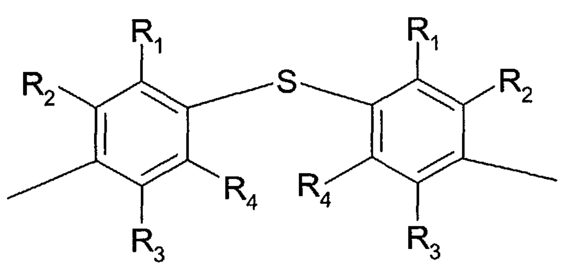

(V)

les paraphénylène vinylène de formule (VI) suivante :

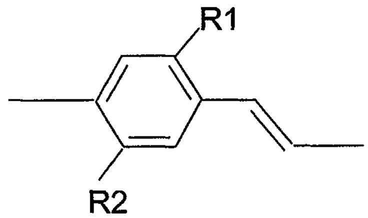

(VI)

les indoles de formule (VII) suivante :

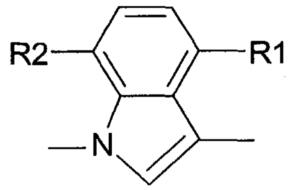

(VII)

les amides aromatiques de formules suivantes (VIIIa), (VIIIb), (VIIIc), (VIIId) :

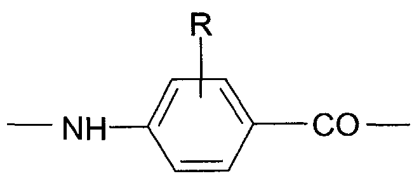

(VIIIa)

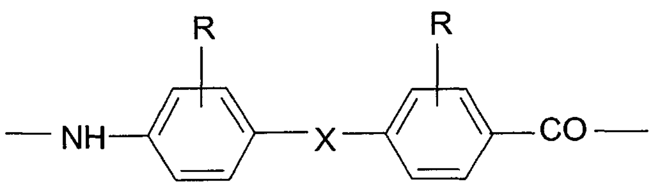

(VIIIb)

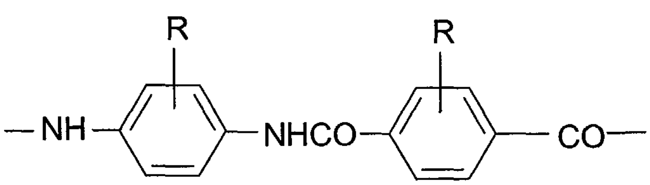

(VIIIc)

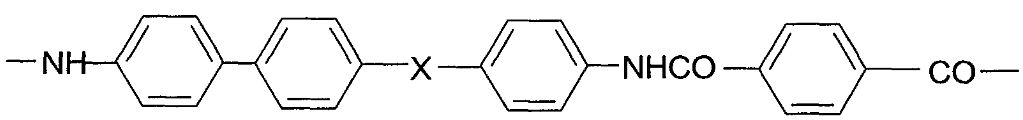

(VIIId)

les hydrazides aromatiques de formules (IXa), (IXb) et (IXc) suivantes :

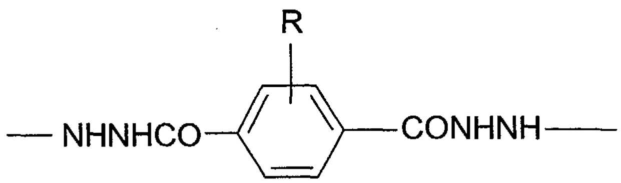

(IXa)

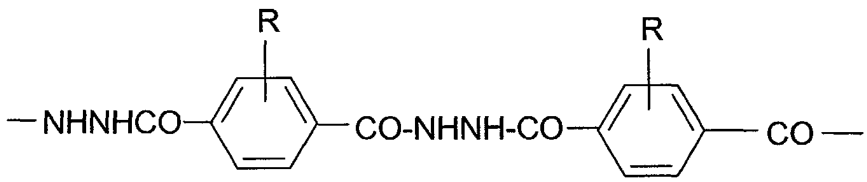

(IXb)

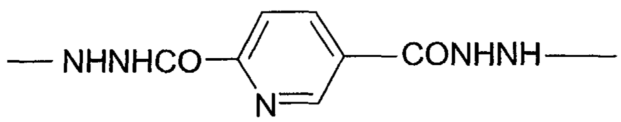

(IXc)

les azométhines aromatiques de formules (Xa), (Xb) et (Xc) suivantes :

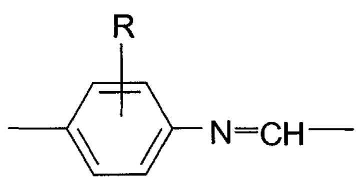

(Xa)

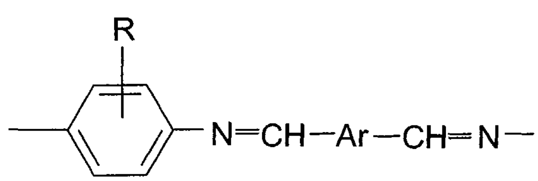

(Xb)

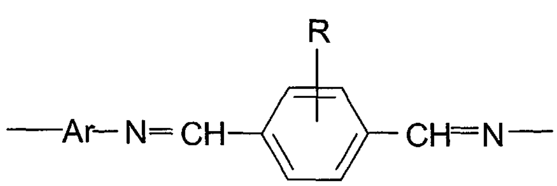

(Xc)

les esters aromatiques de formules (XIa), (XIb) et (XIc) suivantes :

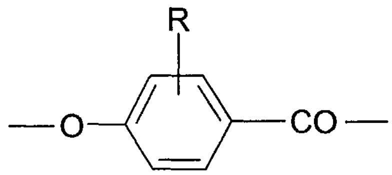

(XIa)

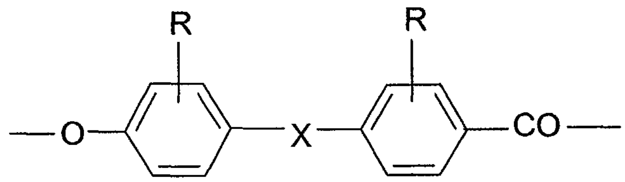

(XIb)

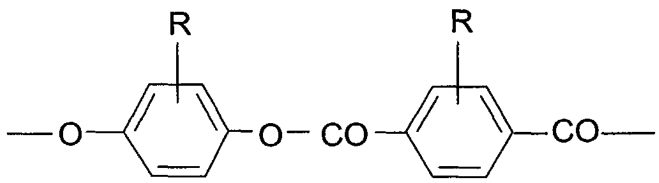

(XIc)

formules (I) à (XI) dans lesquelles :

les radicaux R, R1 à R4 identiques ou différents, sont choisis dans le groupe formé par l'hydrogène, un radical -R', -OR', -COOR', -OCOR', avec R' représentant un radical alkyle linéaire ou ramifié en C1-C20, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, -SO2-, -N=N-, -C(CH3)2-, -CH2-, -CH=CH-, -CH=N- ;
Z = —CH=CH— ou —C≡C—.

**3.** Composition selon l'une des revendications 1 ou 2, **caractérisée en ce que** les groupements solubilisants sont choisis dans le groupe formé par :

- un radical carboxylique (-COOH), carboxylate ($-COO^-M^+$ avec M représentant un métal alcalin, un métal alcalino-terreux, une amine organique, une alcanolamine, un acide aminé),
- un radical sulfonique (-SO3H), sulfonate ($-SO_3^-$ $M^+$, M ayant la même définition que ci-dessus),
- un radical amine primaire, secondaire, tertiaire,
- un radical ammonium quaternaire tel $-NR'3^+$ $Z^-$ avec Z= Br, Cl, alkyl(C1-C4)-OSO3 et R' alkyles identiques ou non, linéaires ou ramifiés en C1 à C20, ou formant un hétérocycle avec l'azote pour deux d'entre eux,
- un radical hydroxyle,
- un radical polyoxyde d'alkylène en C2-C3.

**4.** Composition selon l'une quelconque des revendications précédentes, **caractérisée en ce que** les groupements solubilisants sont reliés au cycle par l'intermédiaire d'un groupement espaceur tel que par exemple un radical -R"-, -OR"-, - OCOR"- ou -COOR"- avec R" représentant un radical alkyle, linéaire ou ramifié en $C_1$-$C_{20}$, comprenant éventuellement un ou plusieurs hétéroatomes.

**5.** Composition selon l'une quelconque des revendications précédentes, **caractérisée en ce que** les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : $-COOH$, $-CH_2COOH$, $-CH_2OH$, $-(CH_2)_6OH$, $-(CH_2)_3SO_3H$, $-O(CH_2)_3SO_3H$, - $O(CH_2)_3N(CH_2CH_3)_2$, $-[(CH_2)_2O]_xCH_2CH_2OH$, $-[(CH_2)_2O]_xCH_2CH_2OCH_3$ avec x nombre moyen compris entre 0 et 200.

**6.** Composition selon l'une quelconque des revendications 1 à 4, **caractérisée en ce que** le polymère conducteur est tel qu'au moins un radical parmi R, R1 à R4 désigne un groupement solubilisant.

**7.** Composition selon l'une quelconque des revendications précédentes, **caractérisée en ce que** le polymère conducteur comporte au moins un groupement solubilisant par unité répétitive.

**8.** Composition selon l'une quelconque des revendications précédentes, **caractérisée en ce que** les groupements solubilisants sont choisis parmi les groupements acide carboxylique ; acide sulfonique ; les radicaux amine tertiaire ; ammonium quaternaire tels que $-NR'_3{}^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels ; les fonctions acides carboxyliques ou sulfoniques peuvent ou non être neutralisées.

**9.** Composition selon l'une des revendications précédentes, **caractérisé en ce que** le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

**10.** Composition selon l'une quelconque des revendications précédentes, **caractérisée en ce que** le ou les polymères conducteurs sont présents dans des proportions d'au moins 0,001 % en poids par rapport au poids total de la composition.

**11.** Composition selon l'une quelconque des revendications précédentes, **caractérisée en ce que** le ou les polymères conducteurs sont présents dans des proportions d'au plus 50 % en poids par rapport au poids total de la composition.

**12.** Composition selon l'une quelconque des revendications précédentes, **caractérisée en ce que** le teneur en polymère conducteur représente 0,1 à 50 % en poids par rapport au poids total de la composition.

**13.** Composition selon l'une quelconque des revendications précédentes, **caractérisée en ce que** le colorant d'oxydation comprend au moins une base d'oxydation choisie parmi les ortho- et para-phénylènediamines, les bases doubles, les ortho- et para-aminophénols, les bases hétérocycliques suivantes ainsi que leurs sels d'addition avec un acide.

**14.** Composition selon la revendication 13, **caractérisée en ce que** la teneur en base d'oxydation représente de 0,0005 à 12 % en poids, par rapport au poids de la composition tinctoriale.

**15.** Composition selon l'une quelconque des revendications précédentes, **caractérisée en ce que** le colorant d'oxydation comprend au moins un coupleur choisi parmi les méta-aminophénols, les méta-phénylènediamines, les métadiphénols, les naphtols et les coupleurs hétérocycliques et leurs sels d'addition avec un acide.

**16.** Composition selon la revendication 15, **caractérisée en ce que** la teneur en coupleur représente de 0,0001 à 10 % en poids, par rapport au poids de la composition tinctoriale.

**17.** Composition selon l'une quelconque des revendications précédentes, **caractérisée en ce que** le milieu cosmétiquement acceptable est l'eau ou un mélange eau-solvant.

**18.** Composition selon la revendication 17, **caractérisée en ce que** le ou les solvants sont choisis parmi des alcools en C1-C4, des alcools aromatiques, des glycols, des éthers de glycol, des polyols et leurs mélanges.

**19.** Composition selon l'une quelconque des revendications précédentes, **caractérisée en ce qu'**elle comprend en outre au moins un agent tensioactif non ionique, anionique, cationique, amphotère ou zwittérionique.

**20.** Composition prête à l'emploi, **caractérisée en ce qu'**elle comprend la composition selon l'une quelconque des revendications précédentes, et au moins un agent oxydant.

**21.** Composition selon la revendication précédente, **caractérisée en ce que** l'agent oxydant est choisi parmi le peroxyde d'hydrogène, le peroxyde d'urée, les bromates ou ferricyanures de métaux alcalins, les persels tels que les perborates et persulfates, et les enzymes.

**22.** Procédé de traitement des fibres kératiniques humaines et plus particulièrement les cheveux, **caractérisé en ce qu'**il consiste à effectuer les étapes suivantes :

a) on applique une composition prête à l'emploi selon l'une des revendications 20 ou 21, sur les fibres sèches ou humides, et on laisse pauser pendant une durée suffisante pour développer la coloration,
b) on rince éventuellement les fibres,
c) on lave les fibres et on les rince,
d) on sèche ou on laisse sécher les fibres.

**23.** Procédé selon la revendication précédente, **caractérisé en ce que** la composition prête à l'emploi est obtenue par mélange extemporané, avant l'application sur les fibres, d'une composition selon l'une quelconque des revendications 1 à 19, avec une composition oxydante, comprenant dans un milieu approprié pour la teinture, au moins un agent oxydant.

**24.** Procédé selon la revendication 22, **caractérisé en ce que** la composition prête à l'emploi est obtenue en appliquant successivement, dans un ordre ou dans l'autre, une composition selon l'une quelconque des revendications 1 à 19 et une composition oxydante, comprenant dans un milieu approprié pour la teinture, au moins un agent oxydant.

**25.** Utilisation d'une composition comprenant, dans un milieu cosmétiquement acceptable, au moins un polymère conducteur et au moins un colorant d'oxydation, selon l'une des revendications 1 à 21, pour le traitement de fibres kératiniques, dans le but de leur conférer un effet optique.

**26.** Utilisation selon la revendication précédente, **caractérisée en ce que** l'effet optique est un effet de brillance.